(19) Europäisches Patentamt
European Patent Office
Office européen des brevets

(11) Veröffentlichungsnummer: **0 405 264 A1**

(12) # EUROPÄISCHE PATENTANMELDUNG

(21) Anmeldenummer: **90111388.6**

(22) Anmeldetag: **16.06.90**

(51) Int. Cl.⁵: **C07D 249/08**, C07D 233/60, C07D 409/06, A01N 43/653, A01N 43/50

(30) Priorität: **28.06.89 DE 3921163**

(43) Veröffentlichungstag der Anmeldung: **02.01.91 Patentblatt 91/01**

(84) Benannte Vertragsstaaten: **BE CH DE FR GB IT LI NL**

(71) Anmelder: **BAYER AG**

**D-5090 Leverkusen 1 Bayerwerk(DE)**

(72) Erfinder: **Jautelat, Manfred, Dr.**
**Müllersbaum 28**
**D-5093 Burscheid(DE)**
Erfinder: **Brandes, Wilhelm, Dr.**
**Eichendorffstrasse 3**
**D-5653 Leichlingen 1(DE)**
Erfinder: **Dutzmann, Stefan, Dr.**
**Leinenweberweg 33**
**D-4000 Düsseldorf 13(DE)**
Erfinder: **Hänssler, Gerd, Dr.**
**Am Arenzberg 58a**
**D-5090 Leverkusen 3(DE)**

(54) **Hydroxy-keto-azole.**

(57) Neue Hydroxy-keto-azole der Formel

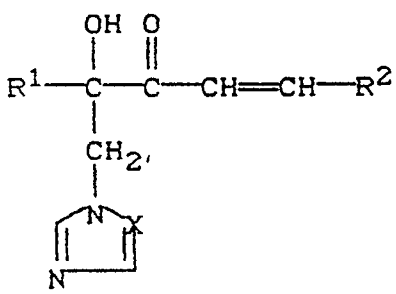

(I)

in welcher
$R^1$ für gegebenenfalls substituiertes Alkyl, gegebenenfalls substituiertes Alkenyl, gegebenenfalls substituiertes Cycloalkyl oder gegebenenfalls substituiertes Aryl steht,
$R^2$ für gegebenenfalls substituiertes Aryl oder für einen gegebenenfalls substituierten und gegebenenfalls benzannellierten, fünf- oder sechsgliedrigen heterocyclischen Rest mit bis zu 3 Heteroatomen steht und
X für ein Stickstoffatom oder eine CH-Gruppe steht, sowie deren Säureadditions-Salze und Metallsalz-Komplexe, ein Verfahren zur Herstellung der neuen Stoffe und deren Verwendung als Mikrobizide im Pflanzenschutz und im Materialschutz.

EP 0 405 264 A1

# HYDROXY-KETO-AZOLE

Die vorliegende Erfindung betrifft neue Hydroxy-keto-azole, ein Verfahren zu deren Hersellung und deren Verwendung als Mikrobizide im Pflanzenschutz und im Materialschutz.

Es ist bereits bekannt, daß sich zahlreiche Hydroxy-ethyl-azolyl-Derivate zur Bekämpfung von Pilzen verwenden lassen (vgl. EP-OS 0 040 345). So können zum Beispiel 1-(2-Chlor-phenyl)-3-(1,2,4-triazol-1-yl-methyl)-4,4-dimethyl-pent-1-en-3-ol, 1-(4-Fluor-phenyl)-3-(1,2,4-triazol-1-yl-methyl)-4,4-dimethyl-pent-1-en-3-ol und 1-(4-Methyl-phenyl)-3-(1,2,4-triazol-1-yl-methyl)-4,4-dimethyl-pent-1-en-3-ol als Fungizide gegen phytopathogene Pilze eingesetzt werden. Bei niedrigen Aufwandmengen läßt die Wirksamkeit dieser Stoffe allerdings in manchen Fällen zu wünschen übrig.

Es wurden nun neue Hydroxy-keto-azole der Formel

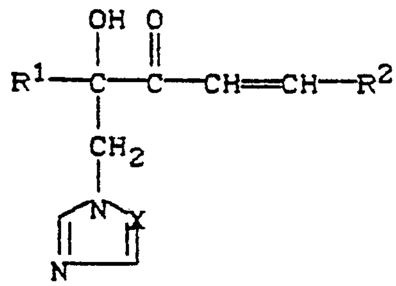

(I)

in welcher

$R^1$ für gegebenenfalls substituiertes Alkyl, gegebenenfalls substituiertes Alkenyl, gegebenenfalls substituiertes Cycloalkyl oder gegebenenfalls substituiertes Aryl steht,

$R^2$ für gegebenenfalls substituiertes Aryl oder für einen gegebenenfalls substituierten und gegebenenfalls benzannellierten, fünf- oder sechsgliedrigen heterocyclischen Rest mit bis zu 3 Heteroatomen steht und

X für ein Stickstoffatom oder eine CH-Gruppe steht,

sowie deren Säureadditions-Salze und Metallsalz-Komplexe gefunden.

Die Verbindungen der Formel (I) enthalten ein asymmetrisch substituiertes Kohlenstoffatom und können deshalb in den beiden optischen Isomerenformen anfallen.

Außerdem können die Stoffe der Formel (I) je nach der Stellung der Wasserstoffatome an der Doppelbindung in zwei geometrischen Isomerenformen vorliegen. Die vorliegende Erfindung betrifft sowohl die Isomerengemische als auch die einzelnen Isomeren.

Weiterhin wurde gefunden, daß man Hydroxy-keto-azole der Formel (I) sowie deren Säureadditions-Salze und Metallsalz-Komplexe erhält, wenn man in einer ersten Stufe Hydroxyalkine der Formel

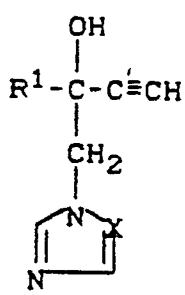

(II)

in welcher

$R^1$ und X die oben angegebene Bedeutung haben,

mit Wasser in Gegenwart von Säuren und/oder Metallkatalysatoren sowie gegebenenfalls in Gegenwart eines Verdünnungsmittels umsetzt und die dabei erhaltenen Hydroxyketone der Formel

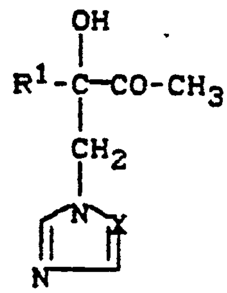

(III)

in welcher
$R^1$ und X die oben angegebene Bedeutung haben,
in einer zweiten Stufe mit Aldehyden der Formel $R^2$-CHO (IV)
in welcher
$R^2$ die oben angegebene Bedeutung hat,
in Gegenwart eines Verdünnungsmittels sowie gegebenenfalls in Gegenwart eines Katalysators umsetzt und gegebenenfalls anschließend an die so erhaltenen Verbindungen der Formel (I) eine Säure oder ein Metallsalz addiert.

Außerdem wurde gefunden, daß die neuen Hydroxy-ketoazole der Formel (I) sowie deren Säureadditions-Salze und Metallsalz-Komplexe sehr gute mikrobizide Eigenschaften besitzen und sowohl im Pflanzenschutz als auch im Materialschutz eingesetzt werden können.

Überraschenderweise zeigen die erfindungsgemaßen Stoffe bei der Bekämpfung von phytopathogenen Pilzen eine deutlich bessere Wirksamkeit als 1-(2-Chlor-phenyl)-3-(1,2,4-triazol-1-yl-methyl)-4,4-dimethyl-pent-1-en-3-ol, 1-(4-Fluorphenyl )-3-(1,2,4-triazol-1-yl-methyl)-4-4-dimethyl-pent-1-en-3-ol und 1-(4-Methyl-phenyl)-3-(1,2,4-triazol-1-yl-methyl)-4,4-dimethyl-pent-1-en-3-ol, welches konstitutionell ähnliche, vorbekannte Wirkstoffe gleicher Wirkungsrichtung sind.

Die erfindungsgemäßen Hydroxy-keto-azole sind durch die Formel (I) allgemein definiert. In dieser Formel stehen vorzugsweise
$R^1$ für geradkettiges oder verzweigtes Alkyl mit 3 bis 7 Kohlenstoffatomen, wobei jeder dieser Reste substituiert sein kann durch Halogen, Cycloalkyl mit 3 bis 7 Kohlenstoffatomen, Phenyl und/oder Halogenphenyl, für gegebenenfalls durch Halogen, Phenyl und/oder Halogenphenyl substituiertes Alkenyl mit 3 bis 6 Kohlenstoffatomen, ferner für Cycloalkyl mit 3 bis 7 Kohlenstoffatomen, wobei jeder dieser Reste durch Alkyl mit 1 bis 4 Kohlenstoffatomen substituiert sein kann, oder für Phenyl, das einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
$R^2$ für Phenyl, das einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano, oder
$R^2$ für einen gegebenenfalls benzanellierten fünfgliedrigen Heterocyclus mit 1 bis 3 Stickstoffatomen, für einen gegebenenfalls benzannellierten fünfgliedrigen Heterocyclus mit einem Schwefelatom, für einen gegebenenfalls benzannellierten fünfgliedrigen Heterocyclus mit einem Sauerstoffatom und einem Schwefelatom, für einen gegebenenfalls benzannellierten fünfgliedrigen Heterocyclus mit einem Sauerstoffatom und einem Stickstoffatom, für einen gegebenenfalls benzanellierten fünfgliedrigen Heterocyclus mit einem Schwefelatom und einem Stickstoffatom, für einen gegebenenfalls benzanellierten sechsgliedrigen Heterocyclus mit 1 bis 3 Stick stoffatomen, wobei jeder der zuvor genannten heterocyclischen Reste ein- oder mehrfach, gleichartig oder verschieden substituiert sein kann durch Halogen, Alkyl mit 1 bis 4 Kohlenstoffatomen, Hydroxyalkyl mit 1 bis 4 Kohlenstoffatomen, Hydroxyalkinyl mit 3 bis 8 Kohlenstoffatomen, Alkoxy mit 1 oder 3 Kohlenstoffatomen, Alkoxy mit 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, wie Fluor- oder Chloratomen, Formyl, Dialkoxymethyl mit 1 oder 2 Kohlenstoffatomen in jeder Alkoxygruppe, Acyl mit 2 bis 4 Kohlenstoffatomen, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 3 Kohlenstoffatomen im Alkylteil, Nitro und/oder Cyano, und

X für ein Stickstoffatom oder eine CH-Gruppe.

Besonders bevorzugt sind diejenigen Verbindungen der Formel (I), in denen

$R^1$ für Isopropyl, tert.-Butyl, tert.-Pentyl, 1-Ethyl-1-methyl-propyl, 1,1-Dimethyl-pentyl, 1,1,2-Trimethylpropyl oder 1,1-Dimethyl-prop-2-enyl steht, wobei jeder dieser zuvor genannten Reste substituiert sein kann durch Fluor, Chlor, Brom, Phenyl, Chlorphenyl, Dichlorphenyl, Fluorphenyl und/oder Difluorphenyl, ferner für 1-Methyl-cyclohexyl, Cyclohexyl, 1-Methylcyclopropyl, Cyclopropyl, 1-Methylcyclopentyl, Cyclopentyl oder 1-Ethylcyclopentyl steht, oder für Phenyl steht, das einfach bis dreifach, gleichartig oder verschieden substitiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Methoxycarbonyl, Ethoxycarbonyl, Methoximinomethyl, 1-Methoximinoethyl, Nitro und/oder Cyano,

$R^2$ für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethylthio, Chlordifluormethoxy, Chlordifluormethylthio, Methoxycarbonyl, Ethoxycarbonyl, Methoximinomethyl, 1-Methoximinoethyl, Nitro und/oder Cyano, oder

$R^2$ für Pyrazolyl, Imidazolyl, 1,2,4-Triazolyl, Pyrrolyl, Furanyl, Thienyl, Thiazolyl, Oxazolyl, Pyridinyl, Pyrimidinyl, Triazinyl, Chinolinyl, Isochinolinyl, Chinazolinyl, Indolyl, Benzothienyl, Benzofuranyl, Benzothiazolyl oder Benzoimidazolyl steht, wobei jeder dieser Reste einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Hydroxymethyl, Hydroxyethyl, Hydroxyalkinyl mit 4 bis 6 Kohlenstoffatomen, Methoxycarbonyl, Ethoxycarbonyl, Methoximinomethyl, 1-Methoximino-ethyl, Nitro, Cyano, Formyl, Dimethoxymethyl, Acetyl und/oder Propionyl, und

X für ein Stickstoffatom oder eine CH-Gruppe steht.

Eine Gruppe ganz besonders bevorzugter erfindungsgemäßer Verbindungen sind diejenigen Stoffe der Formel (I), in denen

$R^1$ für tert.-Butyl, tert.-Pentyl, 1-Ethyl-1-methylpropyl, 1,1,2-Trimethylpropyl, 1,1-Dimethyl-pentyl oder 1,1-Dimethyl-prop-2-enyl steht, wobei jeder dieser Reste ein- oder zweifach durch Fluor und/oder Chlor, durch Phenyl, Chlorphenyl und/oder Fluorphenyl substituiert sein kann, ferner für 1-Methyl-cyclohexyl, 1-Methyl-cyclopentyl oder 1-Ethyl-cyclopentyl steht, oder für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Nitro, Cyano und/oder Methoximinomethyl,

$R^2$ für Phenyl steht, das einfach bis-dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Nitro, Cyano und/oder Methoximinomethyl, oder

$R^2$ für Furanyl, Thienyl, Thiazolyl, Pyridinyl, Pyrimidinyl, Chinolinyl, Benzothienyl, Benzothiazolyl oder Benzofuranyl steht, wobei jeder dieser Reste einfach bis dreifach, gleichartig oder verschieden substituiert sein kann durch Fluor, Chlor, Brom, Methyl, Ethyl, Methoxy, Ethoxy, Methylthio, Trifluormethyl, Trifluormethoxy, Trifluormethylthio, Hydroxymethyl, 3-Hydroxy-3-methylbut-1-in-1-yl, Methoxycarbonyl, Ethoxycarbonyl, Formyl, Dimethoxymethyl, Methoximinomethyl, Methoximino-ethyl, Nitro und/oder Cyano, und

X für ein Sauerstoffatom oder eine CH-Gruppe steht.

Bevorzugte erfindungsgemäße Verbindungen sind auch Additionsprodukte aus Säuren und denjenigen Hydroxy-ketoazolen der Formel (I), in denen $R^1$, $R^2$ und X die Bedeutungen haben, die bereits vorzugsweise für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der

Elemente und denjenigen Hydroxy-keto-azolen der Formel (I), in denen $R^1$, $R^2$ und X die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als spezielle Beispiele für erfindungsgemäße Stoffe seien die in der folgenden Tabelle aufgeführten Hydroxy-keto-azole genannt.

<u>Tabelle 1</u>

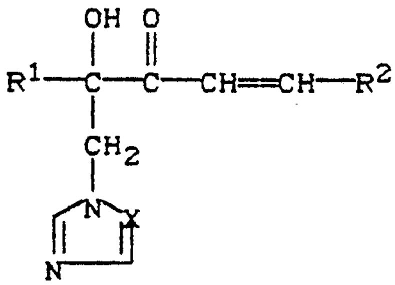

(I)

| $R^1$ | $R^2$ | X |
|---|---|---|
| 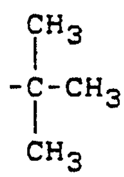 | 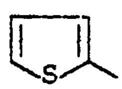 | N |
| 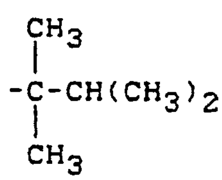 | " | N |
| 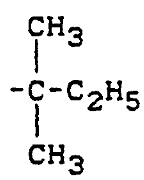 | " | N |
| 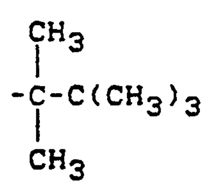 | " | N |

Tabelle 1 (Fortsetzung)

| $R^1$ | $R^2$ | X |
|---|---|---|
| 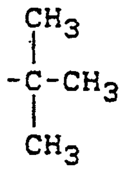 | 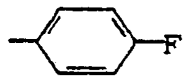 | N |
| 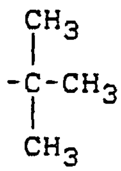 | 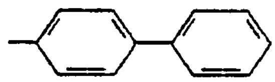 | N |
| 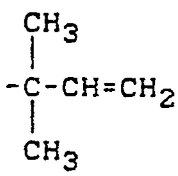 | 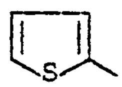 | N |
| 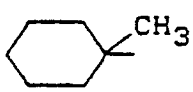 | 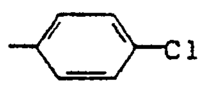 | N |
| 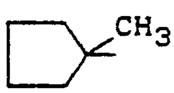 | " | N |
| 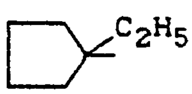 | " | N |
| 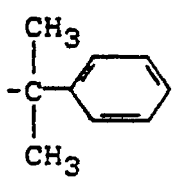 | " | N |

Tabelle 1 (Fortsetzung)

| $R^1$ | $R^2$ | X |
|---|---|---|
| 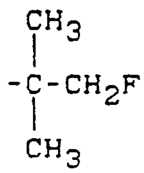 | 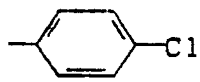 | N |
| 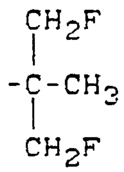 | " | N |
| 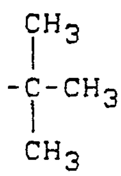 | " | CH |
| 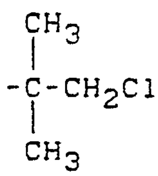 | " | N |
| 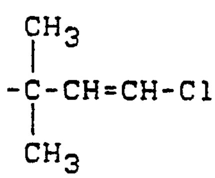 | " | N |
| 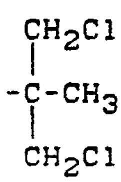 | " | N |

Tabelle 1 (Fortsetzung)

| $R^1$ | $R^2$ | X |
|---|---|---|
| 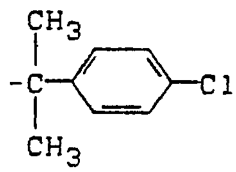 | 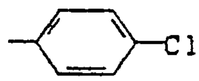 | N |
| 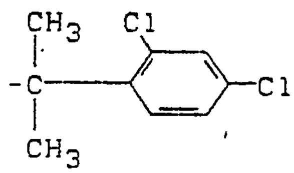 | " | N |
| 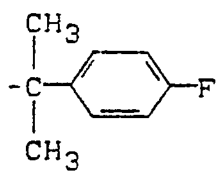 | " | N |
| 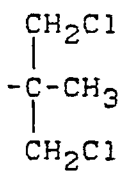 | " | CH |
| 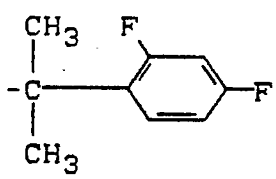 | " | N |
| 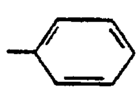 | " | N |

Tabelle 1 (Fortsetzung)

| $R^1$ | $R^2$ | X |
|---|---|---|
| 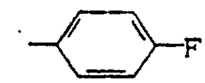 | 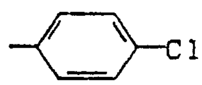 | N |
| 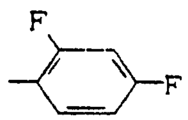 | " | N |
| 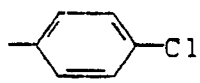 | " | CH |
| 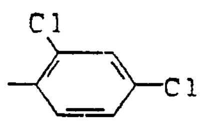 | " | N |
| 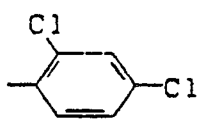 | " | CH |
| 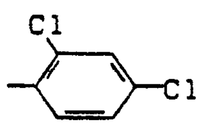 | 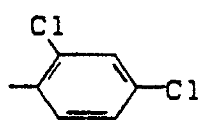 | CH |
| 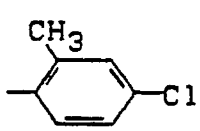 | 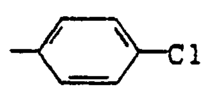 | N |

Tabelle 1 (Fortsetzung)

| $R^1$ | $R^2$ | X |
|---|---|---|
| 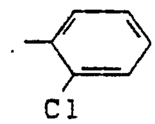 | 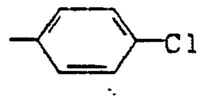 | N |
| 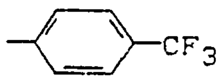 | " | N |
| 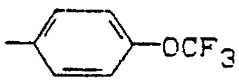 | " | N |
| 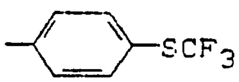 | " | N |
| 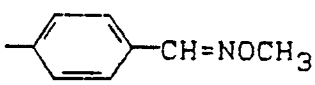 | " | N |
| 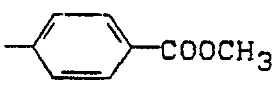 | " | N |
| 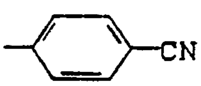 | " | N |
| 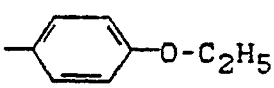 | " | N |

Verwendet man 4,4-Dimethyl-3-hydroxy-3-[(1,2,4-triazol-1-yl)-methyl]-1-pentin als Ausgangssubstanz, Schwefelsäure und Quecksilber(II)-acetat als Reaktionsbeschleuniger in der ersten Stufe und 4-Chlorbenzaldehyd als Reaktionskomponente in der zweiten Stufe, so kann der Verlauf des erfindungsgemäßen Verfahrens durch das folgende Formelschema veranschaulicht werden:

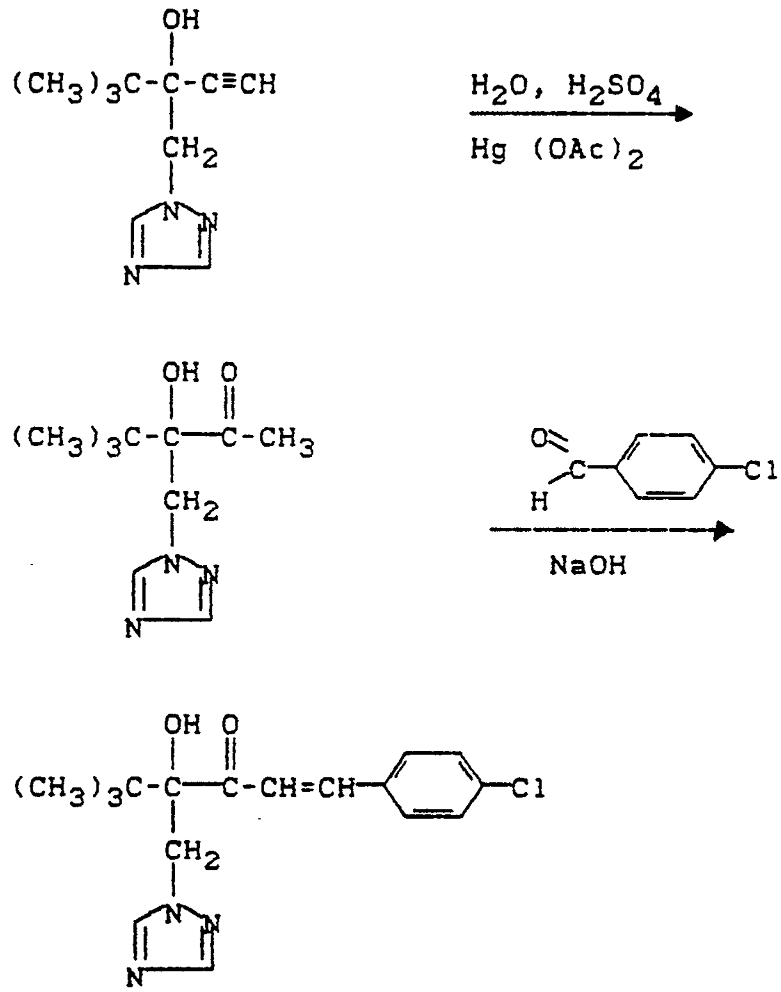


Die bei der Durchführung des erfindungsgemmäßen Verfahrens als Ausgangsstoffe benötigten Hydroxyalkine sind durch die Formel (II) allgemein definiert. In dieser Formel haben $R^1$ und X vorzugsweise diejenigen Bedeutungen, die bereits im Zusammenhang mit der Beschreibung der erfindungsgemäßen Stoffe der Formel (I) vorzugsweise für diese Reste genannt wurden.

Die Hydroxyalkine der Formel (II) sind bereits bekannt (vgl. EP-OS 0 304 552).

Als Reaktionskomponente in der ersten Stufe des erfindungsgemäßen Verfahrens dient Wasser.

Als Reaktionsbeschleuniger kommen bei der Durchführung der ersten Stufe des erfindungsgemäßen Verfahrens alle für derartige Hydratisierungen üblichen Säuren und/oder Metallkatalysatoren in Frage. Vorzugsweise verwendbare Säuren sind Schwefelsäure, Phosphorsäure, Salzsäure, Bromwasserstoffsäure, Salpetersäure, Trichloressigsäure, Trifluoressigsäure sowie auch saure Ionenaustauscherharze. Vorzugsweise verwendbare Metallkatalysatoren sind Schwermetallsalze und Oxide wie Quecksilber(II)-sulfat, Quecksilber(II)-acetat, Quecksilber(II)-chlorid, Quecksilber(II)-oxid, Kupfer(II)-sulfat und Kupfer(I)-chlorid.

Als Verdünnungsmittel kommen bei der Durchführung der ersten Stufe des erfindungsgemäßen Verfahrens Wasser sowie alle üblichen inerten organischen Solventien in Frage.

Vorzugsweise verwendbar sind Wasser, ferner Alkohole, wie Ethanol und Butanol, außerdem Ether, wie Dioxan, weiterhin Säuren wie Essigsäure und darüber hinaus Ketone, wie Aceton. Es können auch Gemische dieser Solventien mit Wasser eingesetzt werden.

Die Reaktionstemperaturen können bei der Durchführung der ersten Stufe des erfindungsgemäßen Verfahrens innerhalb eines größeren Bereiches variiert werden. Im allgemeinen arbeitet man bei Temperaturen zwischen 0 °C und 150 °C, vorzugsweise zwischen 20 °C und 120 °C.

Bei der Durchführung der ersten Stufe des erfindungsgemäßen Verfahrens arbeitet man im allgemeinen unter Normaldruck. Es ist jedoch auch möglich, unter erhöhtem oder vermindertem Druck zu arbeiten.

Bei der Durchführung der ersten Stufe des erfindungsgemäßen Verfahrens setzt man auf 1 Mol an Hydroxyalkin der Formel (II) im allgemeinen 4 bis 10 Mol an Wasser sowie eine katalytische Menge Säure und/oder Metallkytalysator ein. Die Aufarbeitung erfolgt nach üblichen Methoden. Im allgemeinen geht man so vor, daß man das Reaktionsgemisch mit einem in Wasser wenig löslichen organischen Lösungsmittel

verdünnt, mit wäßrig-basischer Lösung wäscht, dann trocknet und einengt und das anfallende Produkt gegebenenfalls weiteren Reinigungsmethoden unterwirft. Die Hydroxyketone der Formel (III) sind bisher noch nicht bekannt.

Die bei der Durchführung der zweiten Stufe des erfindungsgemäßen Verfahrens als Reaktionskomponenten benötigten Aldehyde sind durch die Formel (IV) allgemein definiert in dieser Formel hat $R^2$ vorzugsweise diejenigen Bedeutungen, die bereits im Zusammenhang mit der Beschreibung der erfindungsgemäßen Stoffe der Formel (I) vorzugsweise für diesen Rest genannt wurden.

Die Aldehyde der Formel (IV) sind allgemein bekannte Verbindungen der organischen Chemie.

Als Katalysatoren kommen bei der Durchführung der zweiten Stufe des erfindungsgemäßen Verfahrens alle für derartige Kondensationen üblichen Reaktionsbeschleuniger in Frage. Vorzugsweise verwendbar sind basische Substanzen, z.B. Alkalimetallhydroxide wie Natriumhydroxid und Kaliumhydroxid.

Als Verdünnungsmittel kommen bei der Durchführung der zweiten Stufe des erfindungsgemäßen Verfahrens alle für derartige Umsetzungen üblichen inerten organischen Solventien in Betracht. Vorzugsweise verwendbar sind Alkohole wie Methanol, Ethanol, Isopropanol, n-Butanol und tert.-Butanol sowie Wasser, gegebenenfalls im Gemisch mit Alkoholen.

Die Reaktionstemperaturen können bei der Durchführung der zweiten Stufe des erfindungsgemäßen Verfahrens innerhalb eines größeren Bereiches variiert werden. Im allgemeinen arbeitet man bei Temperaturen zwischen 0° C und 100° C, vorzugsweise zwischen 10° C und 80° C.

Bei der Durchführung der zweiten Stufe des erfindungsgemäßen Verfahrens arbeitet man im allgemeinen unter Normaldruck. Es ist jedoch auch möglich, unter erhöhtem oder vermindertem Druck zu arbeiten.

Bei der Durchführung der zweiten Stufe des erfindungsgemäßen Verfahrens setzt man auf 1 Mol an Hydroxyketon der Formel (III) im allgemeinen 0,5 bis 1,0 Mol an Aldehyd der Formel (IV) sowie eine katalytische Menge an Reaktionsbeschleuniger ein. Die Aufarbeitung erfolgt nach üblichen Methoden. Im allgemeinen geht man in der Weise vor, daß man das Reaktionsgemisch mit einem mit Wasser wenig mischbaren organischen Solvens versetzt, mehrfach mit Wasser wäscht, dann trocknet und einengt. Zur Beseitigung von eventuell noch vorhandenen Verunreinigungen kann das anfallende Produkt weiteren Reinigungsmaßnahmen unterworfen werden.

In einer besonderen Variante kann das erfindungsgemäße Verfahren auch so durchgeführt werden, daß das als Zwischenprodukt auftretende Hydroxyketon der Formel (III) nicht isoliert, sondern direkt weiter umgesetzt wird.

Die nach dem erfindungsgemäßen Verfahren herstellbaren Hydroxy-keto-azole der Formel (I) können in Säureadditions-Salze bzw. Metallsalzkomplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

zur Herstellung von Metallsalz-Komplexen der Verbindungen der Formel (I) kommen vorzugsweise diejenigen Salbe von Metallen in Frage, die bereits im Zusammenhang mit der Beschreibung der erfindungsgemäßen Metallsalz-Komplexe als bevorzugte Metallsalze genannt wurden.

Die Metallsalz-Komplexe von Verbindungen der allgemeinen Formel (I) können in einfacher Weise nach üblichen Verfahren erhalten werden, so z.B durch Lösen des Metallsalzes in Alkohol, z.B Ethanol, und Hinzufügen zu Verbindungen der allgemeinen Formel (I). Man kann Metallsalz-Komplexe in bekannter Weise, z.B durch Abfiltrieren, isolieren und gegebenenfalls durch Umkristallisation reinigen.

Die erfindungsgemäßen Wirkstoffe weisen eine starke mikrobizide Wirkung auf und können als Fungizide im Pflanzenschutz und im Materialschutz eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;

Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Stoffe eignen sich insbesondere zur Bekämpfung von Getreide- und Reiskrankheiten. So lassen sich Mehltau- und Rost-Krankheiten, Puccinia recondita, Leptosphaeria nodorum, Pyrenophora teres, Cochliobolus sativus und Erysiphe graminis an Getreide sowie Pyricularia und Pellicularia an Reis besonders gut bekämpfen. Die Stoffe können außerdem sehr gut gegen Venturia an Äpfeln und gegen Botrytis an Bohnen eingesetzt werden; sie besitzen ferner eine sehr gute invitro-Wirkung.

Im Materialschutz lassen sich die erfindungsgemäßen Wirkstoffe zum Schutz von technischen Materialien einsetzen Unter technischen Materialien sind in diesem Zusammenhang nicht lebende Materialien zu verstehen, die für die Verwendung in der Technik zübereitet worden sind. Beispielsweise können technische Materialien, die durch die erfindungsgemäßen Wirkstoffe vor mikrobieller Veränderung oder Zerstörung geschützt werden sollen, Klebstoffe, Leime, Papier, Karton, Textilien, Leder, Holz, Anstrichmittel, Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Kühlkreisläufe genannt, besonders bevorzugt Holz.

Als Mirkoorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten), sowie gegen Schleimorganismen und Algen. Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puteana
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,

Escherichia, wie Escherichia coli,
Pseudomonas, wie Pseudomonas aeruginosa,
Staphylococcus, wie Staphylococcus aureus.

Die erfindungemäßen Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxy ethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und anderen Wachstumsregulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemaßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von0,0001 bis 0,02 %, am Wirkungsort erforderlich.

Die Herstellung und die Verwendung der erfindungsgemäßen Wirkstoffe wird durch die folgenden Beispiele veranschaulicht.

Herstellungsbeispiele

Beispiel 1

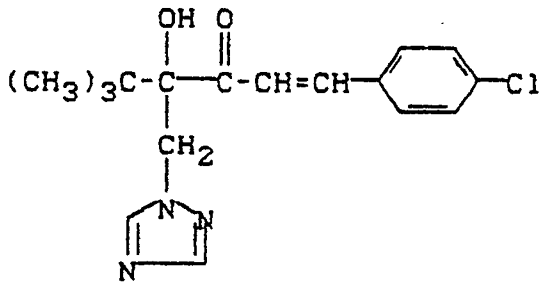

(I-1)

Erste Stufe:

Ein Gemisch aus 19,3 g (0,1 Mol) 4,4-Dimethyl-3-hydroxy3-[(1,2,4-triazol-1-yl)-methyl]-1-pentin, 200 ml Ethanol, 10 ml Wasser 20 ml konzentrierter Schwefelsäure und 5 g Quecksilber(II)-acetat wird 5 Stunden unter Rückfluß erhitzt. Danach läßt man das Reaktionsgemisch auf Raumtemperatur abkühlen, verdünnt mit Methylenchlorid und wäscht mehrfach mit verdünnter, wäßriger Natronlauge. Die organische Phase wird über Natriumsulfat getrocknet und unter vermindertem Druck eingeengt. Man erhält auf diese Weise 20,3 g (80 % der Theorie) an 4,4-Dimethyl-3-hydroxy-[(1,2,4-triazol-1-yl)-methyl]-pentan-2-on der Formel

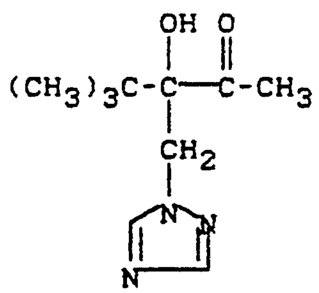

(III-1)

in Form einer Festsubstanz vom Schmelzpunkt 80 bis 83 °C. Nach Umkristallisation aus Toluol weist die Verbindung einen Schmelzpunkt von 96 bis 97 °C auf.
$^1$H-NMR-Spektrum ($CDCl_3$):
$\delta$ = 1,1 (s, 9H); 2,2 (s, 3H); 4,2 (d, J = 13 Hz, 1H);
4,6 (OH), 4,85 (d, J = 13 Hz, 1H); 7,9 (s, 1H);
8,05 (s, 1H)

Zweite Stufe:

12,7 g (60 mmol) 4,4-Dimethyl-3-hydroxy-[(1,2,4-triazol-1-yl)-methyl]-pentan-2-on und 7,1 g (50 mmol) 4-Chlorbenzaldehyd werden in 100 ml Ethanol gelöst und bei 20 °C nach Zugabe einer Lösung von 4 g (0,1 Mol) Natriumhydroxid in 10 ml Wasser 3 Stunden gerührt. Danach wird das Reaktionsgemisch mit Methylenchlorid verdünnt und mehrfach mit Wasser ausgeschüttelt. Die organische Phase wird über Natriumsulfat getrocknet und durch Abziehen des Lösungsmittels unter vermindertem Druck eingeengt. Man erhält auf diese Weise 15,2 g (91 % der Theorie) an 1-(4-Chlorphenyl)-5,5-dimethyl-4-hydroxy-4-[-(1,2,4-triazol-1-yl)-methyl]-hex-1-en-3-on in Form einer Festsubstanz, die nach Umkristallisation aus Methylcyclohexan einen Schmelzpunkt von 83 bis 85 °C aufweist.
$^1$H-NMR-Spektrum ($CDCL_3$):
$\delta$ = 1,1 (s, 9H); 4,35 (d, J = 14 Hz, 1H);
5,0 (d, J = 14 Hz, 1H), 5,0 (OH), 7,3-7,5 (m, 6H); 7,85 (s, 1H);

8,1 (s, 1H)

Nach der zuvor beschriebenen Methode werden auch die in der folgenden Tabelle formelmäßig aufgeführten Verbindungen hergestellt.

Tabelle 2

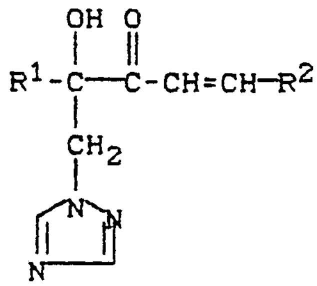

(I)

| Bsp. Nr. | Verb.-Nr. | $R^1$ | $R^2$ | X | Schmelzpunkt (°C) |
|---|---|---|---|---|---|
| 2 | I-2 | $-C(CH_3)_3$ | 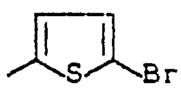 | N | 118 |
| 3 | I-3 | $-C(CH_3)_3$ | 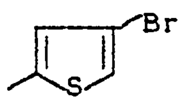 | N | Öl |
| 4 | I-4 | $-C(CH_3)_3$ | 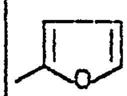 | N | 65-68 |
| 5 | I-5 | $-C(CH_3)_3$ | 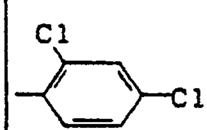 | N | 129-131 |
| 6 | I-6 | $-C(CH_3)_3$ | 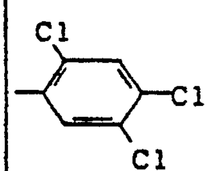 | N | Öl (2-Isomere) |
| 7 | I-7 | 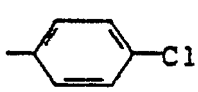 | 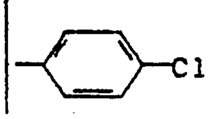 | N | 52-55 |

Verwendungsbeispiele

In den folgenden Verwendungsbeispielen wurden die Verbindungen der nachstehend angegebenen Formeln als Vergleichssubstanzen eingesetzt:

(A) = 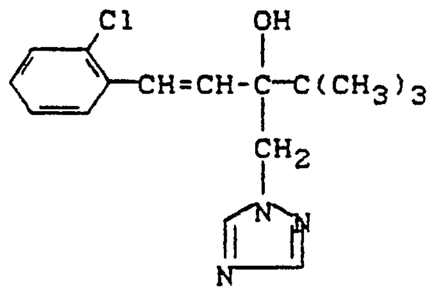

(B) = 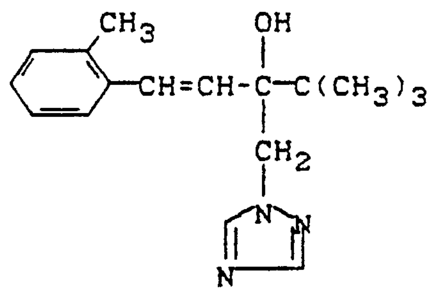

(C) = 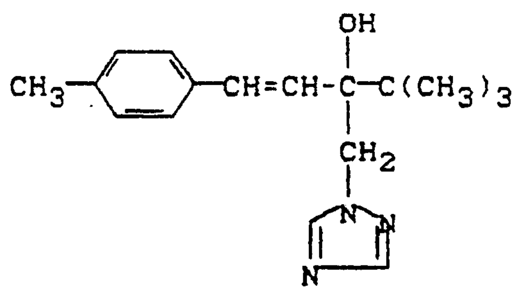

(D) = 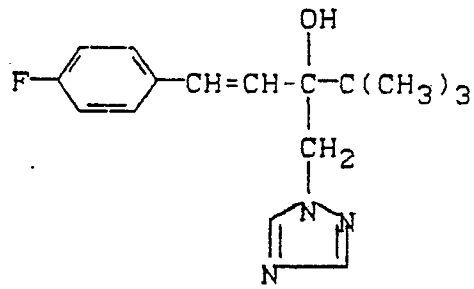

(E) = 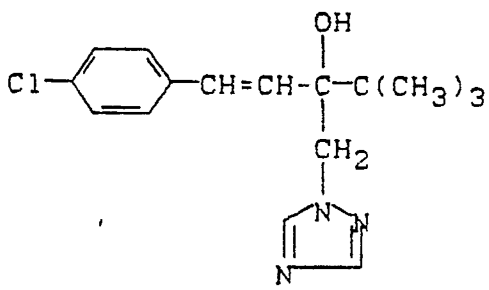

(F) = 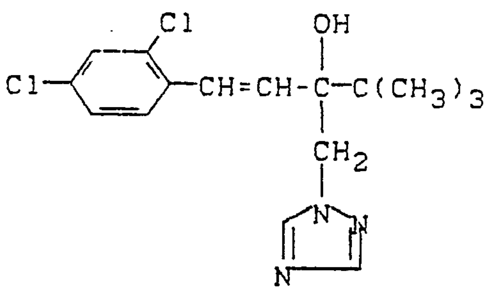

Die Vergleichssubstanzen sind aus der EP-OS 0 040 345 bekannt.

Beispiel A

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzübereitung bis zur Tropfnässe. Nach dem Antrocknen des Spritzbelages werden die Pflanzen mit einer wäßrigen Sporensuspension von Pyricularia oryzae inokuliert. Anschließend werden die Pflanzen in einem Gewächshaus bei 100 % rel Luftfeuchtigkeit und 25° C aufgestellt.

4 Tage nach der Inokulation erfolgt die Auswertung des Krankheitsbefalles.

In diesem Test zeigen die erfindungsgemäßen Verbindungen (I-2), (I-3), (I-4), (I-5) und (I-7) eine wesentlich bessere Wirksamkeit als die Vergleichssubstanz (A).

Beispiel B

Leptosphaeria norodum-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzübereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzübereitung taufeucht. Nach dem Antrocknen des Spritzbelages werden die Pflanzen mit einer Sporensuspension von Leptosphaeria norodum besprüht. Die Pflanzen verbleiben 48 Stunden bei 20° C und 100 % rel. Luftfeuchtigkeit in einer Inkubationskabine.

Die Pflanzen werden in einem Gewächshaus bei einer Temperatur von ca. 15° C und einer relativen Luftfeuchtigkeit von ca. 80 % aufgestellt.

10 Tage nach der Inokulation erfolgt die Auswertung.

In diesem Test zeigen die erf indungsgemäßen Verbindungen (I-1) und (I-3) eine wesentlich bessere Wirkung als die Vergleichssubstanzen (B), (C) und (D).

## Beispiel C

Cochliobolus sativus-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzübereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespüht man junge Pflanzen mit der Wirkstoffzübereitung taufeucht. Nach dem Antrocknen des Spritzbelages werden die Pflanzen mit einer Sporensuspension von Cochliobolus sativus besprüht. Die Pflanzen verbleiben 48 Stunden bei 20° C und 100 % rel. Luftfeuchtigkeit in einer Inkubationskabine.

Die Pflanzen werden in einem Gewächshaus bei einer Temperatur von ca. 20° C und einer relativen Luftfeuchtigkeit von ca. 80 % aufgestellt.

7 Tage nach der Inokulation erfolgt die Auswertung.

In diesem Test zeigen die erfindungsgemäßen Verbindungen (I-1) und (I-3) eine wesentlich bessere Wirkung als die Vergleichssubstanzen (B), (E) und (F).

## Beispiel D

Venturia-Test (Äpfel) /protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Pflanzen mit der Wirkstoffzübereitung bis zur Tropfnässe. Nach dem Antrocknen des Spritzbelages werden die Pflanzen mit einer wäßrigen Konidiensuspension des Apfelschorferregers (Venturia inaequalis) inokuliert und verbleiben dann 1 Tag bis 20° C und 100% rel. Luftfeuchtigkeit in einer Inkubationskabine.

Die Pflanzen werden dann im Gewächshaus bei 20° C und einer relativen Luftfeuchtigkeit von ca. 70 % aufgestellt.

12 Tage nach der Inokulation erfolgt die Auswertung.

In diesem Test zeigt die erfindungsgemäße Verbindung (I-1) eine wesentlich bessere Wirksamkeit als die Vergleichssubstanz (E).

Beispiel E

Botrytis-Test (Bohne) /protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Pflanzen mit der Wirkstoffzubereitung bis zur Tropfnässe. Nach dem Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt.

3 Tage nach der Inokulation wird die Größe der Befallsflecken auf den Blättern ausgewertet.

In diesem Test zeigen die erfindungsgemäßen Verbindungen (I-1), (I-3) und (I-5) eine wesentlich bessere Wirksamkeit als die Vergleichssubstanz (F).

**Ansprüche**

1. Hydroxy-keto-azole der Formel

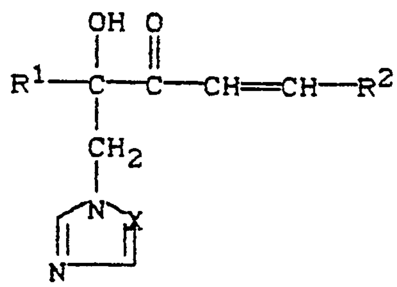

(I)

in welcher
$R^1$ für gegebenenfalls substituiertes Alkyl, gegebenenfalls substituiertes Alkenyl, gegebenenfalls substituiertes Cycloalkyl oder gegebenenfalls substituiertes Aryl steht,
$R^2$ für gegebenenfalls substituiertes Aryl oder für einen gegebenenfalls substituierten und gegebenenfalls benzannellierten, fünf- oder sechsgliedrigen heterocyclischen Rest mit bis zu 3 Heteroatomen steht und
X für ein Stickstoffatom oder eine CH-Gruppe steht,
sowie deren Säureadditions-Salze und Metallsalz-Komplexe.

2. Hydroxy-keto-azole der Formel (I) gemäß Anspruch 1, in denem $R^1$ für geradkettiges oder verzweigtes Alkyl mit 3 bis 7 Kohlenstoffatomen steht, wobei jeder dieser Reste substituiert sein kann durch Halogen, Cycloalkyl mit 3 bis 7 Kohlenstoffatomen, Phenyl und/oder Halogenphenyl, für gegebenenfalls durch Halogen, Phenyl und/oder Halogenphenyl substituiertes Alkenyl mit 3 bis 6 Kohlenstoffatomen steht, ferner für Cycloalkyl mit 3 bis 7 Kohlenstoffatomen steht, wobei jeder dieser Reste durch Alkyl mit 1 bis 4 Kohlenstoffatomen substituiert sein kann, oder für Phenyl steht, das einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
$R^2$ für Phenyl steht, das einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen

Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano, oder

$R^2$ für einen gegebenenfalls benzanellierten fünfgliedrigen Heterocyclus mit 1 bis 3 Stickstoffatomen, für einen gegebenenfalls benzannellierten fünfgliedrigen Heterocyclus mit einem Schwefelatom, für einen gegebenenfalls benzannellierten fünfgliedrigen Heterocyclus mit einem Sauerstoffatom und einem Schwefelatom, für einen gegebenenfalls benzannellierten fünfgliedrigen Heterocyclus mit einem Sauerstoffatom und einem Stickstoffatom, für einen gegebenenfalls benzanellierten fünfgliedrigen Heterocyclus mit einem Schwefelatom und einem Stickstoffatom, oder für einen gegebenenfalls benzanellierten sechsgliedrigen Heterocyclus mit 1 bis 3 Stickstoffatomen steht, wobei jeder der zuvor genannten heterocyclischen Reste ein-oder mehrfach, gleichartig oder verschieden substituiert sein kann durch Halogen, Alkyl mit 1 bis 4 Kohlenstoffatomen, Hydroxyalkyl mit 1 bis 4 Kohlenstoffatomen, Hydroxyalkinyl mit 3 bis 8 Kohlenstoffatomen, Alkoxy mit 1 oder 2 Kohlenstoffatomen, Alkylthio mit 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, Formyl, Dialkoxymethyl mit 1 oder 2 Kohlenstoffatomen in jeder Alkoxygruppe, Acyl mit 2 bis 4 Kohlenstoffatomen, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 3 Kohlenstoffatomen im Alkylteil, Nitro und/oder Cyano, und

X für ein Stickstoffatom oder eine CH-Gruppe steht.

3. Hydroxy-keto-azole der Formel (I) gemäß Anspruch 1, in denen

$R^1$ für Isopropyl, tert.-Butyl, tert.-Pentyl, 1-Ethyl-1-methyl-propyl, 1,1-Dimethyl-pentyl, 1,1,2-Trimethylpropyl oder 1,1-Dimethyl-prop-2-enyl steht, wobei jeder dieser zuvor genannten Reste substituiert sein kann durch Fluor, Chlor, Brom, Phenyl, Chlorphenyl, Dichlorphenyl, Fluorphenyl und/oder Difluorphenyl, ferner für 1-Methyl-cyclohexyl, Cyclohexyl, 1-Methylcyclopropyl, Cyclopropyl, 1-Methyl-cyclopentyl, Cyclopentyl oder 1-Ethyl-cyclopentyl steht, oder für Phenyl steht, das einfach bis dreifach, gleichartig oder verschieden substitiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Methoxycarbonyl, Ethoxycarbonyl, Methoximinomethyl, 1-Methoximinoethyl, Nitro und/oder Cyano,

$R^2$ für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethylthio, Chlordifluormethoxy, Chlordifluormethylthio, Methoxycarbonyl, Ethoxycarbonyl, Methoximinomethyl, 1-Methoximinoethyl, Nitro und/oder Cyano, oder $R^2$ für Pyrazolyl, Imidazolyl, 1,2,4-Triazolyl, Pyrrolyl, Furanyl, Thienyl, Thiazolyl, Oxazolyl, Pyridinyl, Pyrimidinyl, Triazinyl, Chinolinyl, Isochinolinyl, Chinazolinyl, Indolyl, Benzothienyl, Benzofuranyl, Benzothiazolyl oder Benzoimidazolyl steht, wobei jeder dieser Reste einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Hydroxymethyl, Hydroxyethyl, Hydroxyalkinyl mit 4 bis 6 Kohlenstoffatomen, Methoxycarbonyl, Ethoxycarbonyl, Methoximinomethyl, 1-Methoximino-ethyl, Nitro, Cyano, Formyl, Dimethoxymethyl, Acetyl und/oder Propionyl, und

X für ein Stickstoffatom oder eine CH-Gruppe steht.

4. Verfahren zur Herstellung von Hydroxy-keto-azolen der Formel

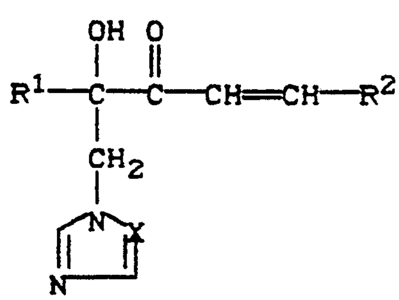

(I)

in welcher

$R^1$ für gegebenenfalls substituiertes Alkyl, gegebenenfalls substituiertes Alkenyl, gegebenenfalls substituiertes Cycloalkyl oder gegebenenfalls substituiertes Aryl steht,

$R^2$ für gegebenenfalls substituiertes Aryl oder für einen gegebenenfalls substituierten und gegebenenfalls benzannellierten, fünf- oder sechsgliedrigen heterocyclischen Rest mit bis zu 3 Heteroatomen steht und

X für ein Stickstoffatom oder eine CH-Gruppe steht,
sowie von deren Säureadditions-Salzen und Metallsalz-Komplexen, dadurch gekennzeichnet, daß man in einer ersten Stufe Hydroxyalkine der Formel

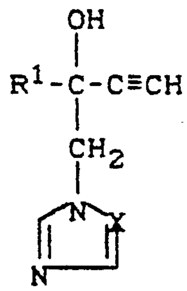

(II)

in welcher
$R^1$ und X die oben angegebene Bedeutung haben,
mit Wasser in Gegenwart von Säuren und/oder Metallkatalysatoren sowie gegebenenfalls in Gegenwart eines Verdünnungsmittels umsetzt und die dabei erhaltenen Hydroxyketone der Formel

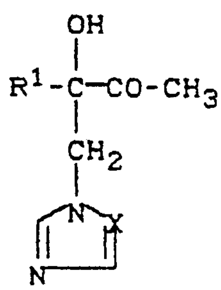

(III)

in welcher
$R^1$ und X die oben angegebene Bedeutung haben,
in einer zweiten Stufe mit Aldehyden der Formel $R^2$-CHO (IV)
in welcher
$R^2$ die oben angegebene Bedeutung hat,
in Gegenwart eines Verdünnungsmittels sowie gegebenenfalls in Gegenwart eines Katalysators umsetzt und gegebenenfalls anschließend an die so erhaltenen Verbindungen der Formel (I) eine Säure oder ein Metallsalz addiert.

5. Mikrobizide Mittel, gekennzeichnet durch einen Gehalt an mindestens einem Hydroxy-keto-azol der Formel (I) gemäß Anspruch 1 bzw. an einem Säure-additons-Salz oder Metallsalz-Komplex eines Hydroxy-keto-azoles der Formel (I).

6. Verwendung von Hydroxy-keto-azolen der Formel (I) gemäß Anspruch 1 bzw. von deren Säureadditions-Salzen und Metallkomplexen als Mikrobizide im Pflanzenschutz und im Materialschutz.

7. Verfahren zur Bekämpfung von unerwünschten Mikroorganismen im Pflanzenschutz und im Materialschutz, dadurch gekennzeichnet, daß man Hydroxy-keto-azole der Formel (I) gemäß Anspruch 1 bzw. deren Säure-additions-Salze oder Metallsalzkomplexe auf die Mikroorganismen und/oder deren Lebensraum ausbringt.

8. Verfahren zur Herstellung von mikrobiziden Mitteln, dadurch gekennzeichnet, daß man Hydroxy-keto-azole der Formel (I) gemäß Anspruch 1 bzw. deren Säureadditionssalze oder Metallsalzkomplexe mit Streckmitteln und/oder oberflächenaktiven Stoffen vermischt. 9. Hydroxyketone der Formel

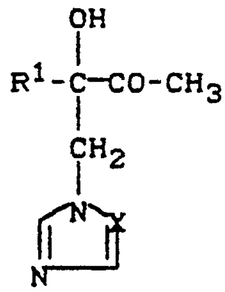

(III)

in welcher

$R^1$ für gegebenenfalls substituiertes Alkyl, gegebenenfalls substituiertes Alkenyl, gegebenenfalls substituiertes Cycloalkyl oder gegebenenfalls substituiertes Aryl steht und

X für ein Stickstoffatom oder eine CH-Gruppe steht.

Europäisches Patentamt

# EUROPÄISCHER RECHERCHENBERICHT

Nummer der Anmeldung

EP 90111388.6

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
| X | CHEMICAL ABSTRACTS, Band 106, Nr. 25, 22. Juni 1987 Columbus, Ohio, USA OGATA MASARU et al. "Synthesis and oral antifungal activity of novel azolylpropanolones and related compounds" Seite 655, Spalte 1, Zusammenfassung-Nr. 213 855b & J.Med.Chem. 1987 30(6), 1054-68 -- | 9 | C 07 D 249/08<br>C 07 D 233/60<br>C 07 D 409/06<br>A 01 N 43/653<br>A 01 N 43/50 |
| X | CHEMICAL ABSTRACTS, Band 104, Nr. 9, 3. März 1986 Columbus, Ohio, USA SHIONOGI AND CO. LTD. "Imidazole and triazole fungicides" Seite 278, Spalte 1, Zusammenfassung-Nr. 64 208x & Jpn. Kokai Tokkyo Koho JP 60,172,904 (85,172,904) -- | 9 | RECHERCHIERTE SACHGEBIETE (Int Cl 5)<br>C 07 D 249/00<br>C 07 D 233/00<br>C 07 D 409/00 |
| A | EP - A2 - 0 114 487 (JCJ) * Ansprüche 1,10,12 * -- | 1,5-8 | |
| D,A | EP - A1 - 0 040 345 (BAYER) * Ansprüche 1,4-6 * -- | 1,5-8 | |
| A | EP - A2 - 0 117 578 (SHIONOGI) * Ansprüche 1,3 * -- | 1,5 | |
| A | EP - A1 - 0 304 552 (BAYER) * Ansprüche 1,4-6 * ---- | 1,5 | |

Der vorliegende Recherchenbericht wurde für alle Patentansprüche erstellt.

| Recherchenort | Abschlußdatum der Recherche | Prüfer |
|---|---|---|
| WIEN | 17-09-1990 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82